# EUROPEAN PATENT SPECIFICATION

(11) **EP 3 398 605 B1**
(45) Date of publication and mention of the grant of the patent: **14.07.2021**
(21) Application number: 16881680.9
(22) Date of filing: 21.12.2016
(51) Int. Cl.: A61K 33/00, A61P 9/10, A61P 25/28, A61P 39/06

(54) **MEDICINAL COMPOSITION FOR IMPROVING PROGNOSIS AFTER RESTART OF PATIENT'S OWN HEARTBEAT**
MEDIZINISCHE ZUSAMMENSETZUNG ZUR VERBESSERUNG DER PROGNOSE NACH DEM WIEDERSTARTEN DES EIGENEN HERZSCHLAGS EINES PATIENTEN
COMPOSITION MÉDICINALE POUR AMÉLIORER UN PRONOSTIC APRÈS REDÉMARRAGE DU PROPRE RYTHME CARDIAQUE D'UN PATIENT

(30) Priority: 28.12.2015 JP 2015257318
(43) Date of publication of application: 07.11.2018
(73) Proprietor: Keio University, Tokyo 108-8345 (JP); Taiyo Nippon Sanso Corporation, Tokyo 142-8558 (JP)
(72) Inventor: SUZUKI, Masaru, Tokyo 160-8582 (JP); HAYASHIDA, Kei, Tokyo 160-8582 (JP); TAMURA, Tomoyoshi, Tokyo 160-8582 (JP); SANO, Motoaki, Tokyo 160-8582 (JP); NISHIWAKI, Yoshiki, Tokyo 142-8558 (JP)
(74) Representative: Boult Wade Tennant LLP
(86) International application number: PCT/JP2016/088172
(87) International publication number: WO 2017/115705

(56) References cited:
- WO-A1-2007/021034
- JP-A- 2011 184 398
- YI HUANG ET AL: "Beneficial effects of hydrogen gas against spinal cord ischemia reperfusion injury in rabbits", BRAIN RESEARCH, ELSEVIER, AMSTERDAM, NL, vol. 1378, 23 December 2010 (2010-12-23), pages 125-136, XP028145848, ISSN: 0006-8993, DOI: 10.1016/J.BRAINRES.2010.12.071 [retrieved on 2010-12-31]
- HAYASHIDA K ET AL: "Inhalation of hydrogen gas reduces infarct size in the rat model of myocardial ischemia-reperfusion injury", BIOCHEMICAL AND BIOPHYSICAL RESEARCH COMMUNICATIONS, ELSEVIER, AMSTERDAM, NL, vol. 373, no. 1, 15 August 2008 (2008-08-15), pages 30-35, XP022795076, ISSN: 0006-291X, DOI: 10.1016/J.BBRC.2008.05.165 [retrieved on 2008-06-09]
- NORIKO KANEDA et al.: "Rat Kan Kyoketsu Saikanryu Shogai Model ni Okeru Konodo Suiso Gas no Postconditioning Koka", Bulletin of Japan Research Association for the Medical & Hygienic Use of Ozone, vol. 21, no. 3, 2014, pages 88-94, XP009511787, ISSN: 2186-3784
- OHSAWA, I. et al.: "Hydrogen acts as a therapeutic antioxidant by selectively reducing cytotoxic oxygen radicals", Nature medicine, vol. 13, no. 6, 2007, pages 688-694, XP002700155, ISSN: 1078-8956
- HAYASHIDA, K. et al.: "H2 gas improves functional outcome after cardiac arrest to an extent comparable to therapeutic hypothermia in a rat model", Journal of the American Heart Association, vol. 1, no. 5, 12 October 2012 (2012-10-12), pages 1-13, XP055512240,
- HAYASHIDA, K. et al.: "Hydrogen inhalation during normoxic resuscitation improves neurological outcome in a rat model of cardiac arrest independently of targeted temperature management", Circulation, vol. 130, 16 December 2014 (2014-12-16), pages 2173-2180, XP055512247, ISSN: 0009-7322
- MOTOAKI SANO: "The Potential of Hydrogen Gas as a Novel Medical Gas", Medical Gases, vol. 15, no. 1, 2013, pages 45-48, XP009512421, ISSN: 2434-6152

## Description

### Technical Field]

The present invention relates to a pharmaceutical composition for improving prognosis after return of spontaneous circulation following cardiopulmonary arrest.

### [Background Art]

Patients resuscitated from out-of-hospital cardiac arrest, even if their lives can be saved, have severe aftereffects in the brain or the heart, a low rehabilitation rate, and very poor vital prognosis. According to the multicenter observational study of out-of-hospital cardiac arrest patients conducted in 2012 (58 hospitals in Kanto Area in Japan), 920 resuscitated patients hospitalized after return of spontaneous circulation exhibited a 1-month survival rate of 21.6% and a 1-month consciousness recovery rate of 13.3%. Therapeutic hypothermia, only one treatment found to have efficacy on the organ protection of resuscitated patients, requires a large-scale apparatus and the involvement of many healthcare professionals in charge thereof and is therefore practiced only in some facilities such as university hospitals. Thus, the development of a novel treatment method in place of the therapeutic hypothermia is urgently necessary for improvement of prognosis of resuscitated patients.

Organ damage in the brain, the heart, or the like after cardiac arrest resuscitation is also called post-cardiac arrest syndrome (PCAS) and is considered to involve reactive oxygen species or free radicals generated after return of spontaneous circulation. Hydrogen gas is a selective radical scavenger that eliminates only a hydroxy radical which is a harmful reactive oxygen species, and has a very characteristic property of being able to pass through a cell membrane and reach organelle, irrespective of blood flow, because of its small molecular weight and excellent diffusing power (Non Patent Literature 1). Also, the inhalation of hydrogen gas (49%) is used in the prevention of decompression sickness in humans and known to manifest no adverse reaction even if healthy persons inhale the hydrogen gas (Non Patent Literature 2).

Against this backdrop, the present inventors have found that the inhalation of hydrogen working as a selective radical scavenger can improve brain and heart functions and vital prognosis after cardiopulmonary arrest resuscitation in rats (Non Patent Literatures 3 and 4).

### [Citation List]

### [Non Patent Literature]

[Non Patent Literature 1] Nat Med. 2007 Jun; 13 (6): 688-94
[Non Patent Literature 2] Journal of Applied Physiology March 1, 1994 vol. 76 No. 3 1113-1118
[Non Patent Literature 3] Hayashida K, Sano M, Hori S, et al., Journal of the American Heart Association 2012 Oct 25; 1(5): 1-13; doi: 10.1161/JAHA.112.003459
[Non Patent Literature 4] Hayashida K, et al., Circulation. 2014 Dec 9; 130 (24): 2173-80

### [Summary of Invention]

### [Technical Problem]

The experimental results obtained in rats suggest that hydrogen inhaled from before return of spontaneous circulation to immediately after the return of spontaneous circulation functioned favorably as a selective radical scavenger and reduced damage ascribable to reactive oxygen species after the return of spontaneous circulation. On the other hand, its safety or efficacy in humans is still unknown.

At present, the administration of hydrogen gas to humans requires the consent of patients themselves or their relatives or other persons concerned. Therefore, it is very difficult to carry out hydrogen inhalation in humans from before return of spontaneous circulation to immediately after the return of spontaneous circulation, as in the previous practice in rats. In other words, it is almost impossible to obtain the consent from patients themselves within a period from before return of spontaneous circulation to immediately after the return of spontaneous circulation. Furthermore, a certain amount of time is generally required for obtaining the consent to hydrogen inhalation from persons related to the patients. The generation of reactive oxygen species at a reflow site of blood is considered to occur several minutes after return of spontaneous circulation (Circulation. (2002) 105: 2332-2336; and Proc. Natl. Acad. Sci. USA Vol. 86, pp. 4695-4699 (June 1989)). Hence, the hydrogen inhalation in humans is often started after a lapse of a considerable period of time from the generation of reactive oxygen species in the body. In such a case, effects similar to those previously confirmed in mice cannot be expected because organ damage ascribable to reactive oxygen species has already progressed.

Accordingly, an object of the present invention is to provide a pharmaceutical composition that can improve prognosis after return of spontaneous circulation even after a lapse of a considerable period of time from the return of spontaneous circulation.

### [Solution to Problem]

In the course of studying the safety and efficacy of hydrogen inhalation in patients resuscitated from out-of-hospital cardiac arrest, the present inventors have found that, totally unexpectedly, hydrogen inhalation can significantly improve prognosis after return of spontaneous circulation even in human patients after a lapse of a considerable period of time from the return of circulation following cardiopulmonary arrest. As already mentioned, the effect of hydrogen gas as a selective radical scavenger cannot explain such successful improvement of prognosis after return of spontaneous circulation even in patients in which organ damage ascribable to reactive oxygen species has already progressed. Thus, the findings gained by the present inventors are surprising.

The present invention is based on the findings described above and provides:
[1] A gaseous pharmaceutical composition comprising hydrogen gas for use in treating a human patient who has experienced a cardiopulmonary arrest for at least 10 minutes, wherein the pharmaceutical composition is administered to the patient after a lapse of 30 minutes or longer from the return of spontaneous circulation following the cardiopulmonary arrest, wherein
   the prognosis after the return of spontaneous circulation is improved in the human patient with the administration of the pharmaceutical composition as compared with a human patient group without the administration of the pharmaceutical composition, and wherein
   the improvement of the prognosis is improvement of brain function.
[2] The pharmaceutical composition for use according to [1], wherein
   the human subject is a subject having a Glasgow Coma Scale (GCS) score of 8 or lower.
[3] The pharmaceutical composition for use according to [1] or [2], wherein
   the "improvement of prognosis after return of spontaneous circulation" is improvement at at least 90 days after the return of spontaneous circulation.
[4] The pharmaceutical composition for use according to any of [1] to [3], wherein
   the "improvement of prognosis after return of spontaneous circulation" is determined on the basis of a Cerebral Performance Category (CPC) score.
[5] The pharmaceutical composition for use according to any of [1] to [4], wherein
   the pharmaceutical composition is continuously administered for at least 18 hours from the start of administration.
[6] The pharmaceutical composition for use according to any of [1] to [5], wherein
   the pharmaceutical composition further comprises oxygen gas.
[7] The pharmaceutical composition for use according to any of [1] to [6], wherein
   the pharmaceutical composition further comprises an inert gas.
[8] The pharmaceutical composition for use according to any of [1] to [7], wherein
   the hydrogen concentration in the pharmaceutical composition is 0.1% to 4.0%, optionally wherein
   the hydrogen concentration in the pharmaceutical composition is 1.0% to 2.0%.
[9] The pharmaceutical composition for use according to any of [1] to [8], wherein
   the cardiopulmonary arrest is caused by acute myocardial infarction, cardiomyopathy or hyperkalemia.

Further non-limiting embodiments are described below and set out in the claims.

In accordance with the claims, one of or any combination of two or more of the aspects of the present invention described above is also included in the scope of the present invention.

### [Advantageous Effects of Invention]

The present invention provides a pharmaceutical composition that can improve prognosis after return of spontaneous circulation even after a lapse of a considerable period of time from the return of spontaneous circulation.

### [Description of Embodiments]

Hereinafter, embodiments of the present invention will be specifically described.

The present invention relates to a gaseous pharmaceutical composition comprising hydrogen gas for use, which improves prognosis after return of spontaneous circulation in a human patient who has experienced a cardiopulmonary arrest. The present inventors have newly found that hydrogen gas can improve prognosis after return of spontaneous circulation even in human patients who have experienced organ damage ascribable to free radicals, after a lapse of a considerable period of time from the return of spontaneous circulation following cardiopulmonary arrest. Therefore, the pharmaceutical composition for use of the present invention is administered to a human patient who has experienced a cardiopulmonary arrest for at least 10 minutes, and after a lapse of 30 minutes or longer from subsequent return of spontaneous circulation.

Thus, the pharmaceutical composition for use of the present invention can be administered to a human patient, as a recipient, who has experienced cardiopulmonary arrest over a time long enough to generate injurious reactive oxygen species or free radicals due to return of spontaneous circulation following the cardiopulmonary arrest. In the present invention, such a time is at least 10 minutes. On the other hand, the upper limit of the cardiopulmonary arrest duration is not particularly limited as long as rehabilitation after resuscitation is expected. At present, 35 minutes from cardiopulmonary arrest are used as a guide for terminating resuscitation.

The pharmaceutical composition for use of the present invention can also be administered to a human patient as a recipient after a lapse of a time long enough to generate injurious reactive oxygen species or free radicals after return of spontaneous circulation following cardiopulmonary arrest. In accordance with the claims, such a time is at least 30 minutes, preferably at least 1 hour, at least 2 hours, or at least 3 hours. On the other hand, the upper limit of the time from after the return of spontaneous circulation to the start of administration is not particularly limited. As described below in Examples, the present inventors have suggested, for the first time, that hydrogen gas when administered to human patients after return of spontaneous circulation does not always exert an effect only as a selective radical scavenger. Thus, the pharmaceutical composition for use of the present invention may be administered even after the end of production of reactive oxygen species or free radicals after return of spontaneous circulation.

In the present invention, the cause of the cardiopulmonary arrest is not particularly limited and may be a cardiogenic disease or may be a non-cardiogenic disease. In the present invention, examples of the cardiogenic disease can include, but are not limited to, acute coronary syndromes (ST-elevation myocardial infarction, non-ST-elevation myocardial infarction, unstable angina pectoris, etc.), cardiomyopathy (hypertrophic, dilated, restrictive, arrhythmogenic right ventricular, or unclassified cardiomyopathy), myocarditis, inherited or acquired arrhythmic diseases (long QT syndrome, Brugada syndrome, etc.), preexcitation syndrome, and sick sinus syndrome.

In the present invention, examples of the non-cardiogenic disease can include, but are not limited to, aorta dissection, cardiac tamponade, pulmonary embolism, sepsis, acidosis, hypoxemia, hypercapnia, suffocation, near-drowning, stroke, trauma, massive hemorrhage, tension pneumothorax, hypothermia, hypoglycemia, poisoning (tricyclic antidepressants, opioids, beta blockers, calcium receptor antagonists, digitalis, parasympathomimetic agents, adenosine, adenosine triphosphate, etc.), and electrolyte abnormality (hyperkalemia, etc).

In the present invention, the term "improvement of prognosis" refers to an improvement of brain function. In this respect, the phrase "prognosis after return of spontaneous circulation is improved as compared with a human patient group without the administration of the pharmaceutical composition" includes improvement of the degree of brain function at a certain point in time after the return of spontaneous circulation as compared with the human patient group without the administration of the pharmaceutical composition, and shortening of a time to achieve a given level of improvement in brain function as compared with the human patient group without the administration of the pharmaceutical composition.

In accordance with the claims, the "improvement of prognosis" is an improvement of brain function. The brain function can typically be determined on the basis of a Cerebral Performance Category (CPC) score. The CPC score is an index well known to those skilled in the art, and each score is as described in Table 1 below.

**[Table 1]**

| Cerebral Performance Category (CPC) |
|---|
| (1) CPC 1: Good performance |
| Clear conscious, able to work and lead a normal life. May have minor neurological or psychological deficits such as mild dysarthria, minor cranial nerve disorders, or non-incapacitating hemiparesis. |
| (2) CPC 2: Moderate disability |
| Conscious, sufficient cerebral function for part-time work in sheltered environment or independent activities of daily life such as dress, travel, or food preparation. May have hemiplegia, seizures, ataxia, dysarthria, dysphagia, or permanent memory or mental disability. |
| (3) CPC 3: Severe disability |
| Conscious, dependent on others for daily support because of impaired brain function. Has at least limited cognition. A wide range of cerebral abnormalities from severe memory disturbances or dementia to patients who are paralyzed and can communicate only with their eyes, as in the locked-in syndrome. |
| (4) CPC 4: Coma |
| Unconscious, vegetative state, lack of cognition. Lack of verbal or psychological interaction with environment. |
| (5) CPC 5: Death or brain death |

The pharmaceutical composition for use of the present invention is characterized in that it is a gaseous pharmaceutical composition comprising hydrogen gas. The pharmaceutical composition is also characterized in that it is continuously administered over a predetermined time to a human patient because of being a gaseous pharmaceutical composition. In the present invention, the hydrogen atom may be any of its all isotopes, i.e., protium (P or ¹H), deuterium (D or ²H) , and tritium (T or ³H). Thus, P₂, PD, PT, DT, D₂ and T₂ may be included as molecular hydrogen. In a preferred aspect of the present invention, 99% or more of the hydrogen gas contained in the pharmaceutical composition is natural molecular hydrogen P₂.

The pharmaceutical composition for use of the present invention can further comprise oxygen gas. The oxygen gas may be present in the form of a mixed gas by mixing with the hydrogen gas in advance, or may be mixed with the hydrogen gas immediately before or at the time of administration to a subject.

The pharmaceutical composition for use of the present invention can further comprise an inert gas. The inert gas is used for the purpose of preventing the explosion of the hydrogen gas or the oxygen gas and adjusting the concentration thereof and can thus be present in the form of a mixed gas with the hydrogen gas and/or the oxygen gas. Examples of the inert gas that can be used in the pharmaceutical composition can include, but are not limited to, nitrogen gas, helium gas, and argon gas. In one embodiment of the present invention, inexpensive nitrogen gas is used as the inert gas.

In the pharmaceutical composition for use of the present invention, the concentration range of the hydrogen gas can be, but is not limited to, for example, any concentration from 0.1 to 4.0% (v/v). The lower limit value of the hydrogen gas concentration can be set as the lower limit value of a concentration at which the pharmaceutical composition can exert the effect of improving prognosis after return of spontaneous circulation. Thus, the minimum concentration that permits improvement of prognosis after return of spontaneous circulation can be appropriately set according to the severity of the patient, the cardiopulmonary arrest duration, the time from the return of spontaneous circulation to the start of administration, the type of the disease responsible for cardiac arrest, sex, age, etc. In one embodiment, the lower limit value of the hydrogen gas can be selected as any value from 0.1 to 1.0%, for example, 0.5%. On the other hand, the upper limit value of the hydrogen gas concentration is set from the viewpoint of safety because the lower explosion limit of hydrogen in air is 4%. Thus, the upper limit value of the hydrogen gas can be selected as any concentration of 4% or lower, for example, 3.0%, 2.5% or 2.0% as long as safety is ensured.

In the pharmaceutical composition for use of the present invention, the concentration of the oxygen gas can be in the range of 21% to 99.9% (v/v) on the precondition that the hydrogen gas concentration is 0.1 to 4.0% (v/v).

In the pharmaceutical composition for use of the present invention, the concentration of the inert gas is set in a range in which the concentration of the hydrogen gas and/or the oxygen gas is properly maintained, and the explosion-proof effects of these gases are obtained. Thus, those skilled in the art can appropriately set the concentration of the inert gas to a proper concentration according to the concentration of the hydrogen gas and/or the oxygen gas used. When the inert gas is, for example, nitrogen gas, such a concentration of the inert gas can be arbitrarily adopted in the range of, for example, 0 to 78.9% (v/v).

The concentration of each gas used throughout the present specification is indicated by a content at 20°C at 101.3 kPa.

The pharmaceutical composition for use of the present invention may further comprise other gases such as carbon dioxide in the atmosphere, air, or anesthesia gas, etc. without impairing the effect of the present invention brought about by the hydrogen gas.

The pharmaceutical composition for use of the present invention can be administered to the subject, for example, by inhalation using an inhalation means. Examples of such an inhalation means can include, but are not limited to, inhaling masks. Preferably, the inhaling mask covers the mouth and the nose of the subject at the same time so as to achieve administration at a proper concentration to the subject.

In one embodiment of the present invention, the pharmaceutical composition is provided in a form that can be administered directly to the subject. As one example, in this embodiment, the pharmaceutical composition is provided in the form of a mixed gas prepared by mixing in advance the hydrogen gas and the inert gas, the oxygen gas for respiration, and other arbitrary gasses at proper concentrations.

In another aspect of the present invention, the pharmaceutical composition is provided in a form that is prepared immediately before or at the time of administration to the subject. As one example, in this embodiment, the pharmaceutical composition is provided by connecting a container containing a mixed gas of the hydrogen gas and the inert gas and a container containing the oxygen gas to an inhaling mask via piping, and sending these gases to the patient at a flow rate that attains their concentrations proper for administration to the subject. In one aspect of the present invention, the container may be in the form of a portable gas cylinder as well as, for example, a large storage tank installed outdoors. The gas may be contained in the form of a compressed gas in the container or may be contained in a liquified form in a liquid gas container (LGC). As another example, in the present invention, the hydrogen gas, the oxygen gas, and the inert gas such as nitrogen gas may each be supplied from a gas generation apparatus. Examples of such a generation apparatus include, but are not limited to, oxygen concentrator for oxygen gas, and hydrogen generation apparatuses based on water electrolysis for hydrogen gas.

In an alternative aspect of the present invention, the pharmaceutical composition is provided by supplying the hydrogen gas to a sealed chamber such that the concentration of the gas is kept constant. As one example, in this embodiment, the pharmaceutical composition is provided by supplying, to a sealed chamber where the subject is present, a mixed gas of the hydrogen gas and the inert gas at a flow rate that keeps the hydrogen concentration at a proper concentration in the sealed chamber.

The pharmaceutical composition for use of the present invention can be provided in the form of one or more gas-containing containers or in the form of a device for gas inhalation comprising: a plurality of gas-containing containers; an inhalation means for gas inhalation; and piping which connects the plurality of containers to the gas inhalation means. In one embodiment of the present invention, preferably, the device for gas inhalation comprises a control mechanism which controls a gas flow rate to the gas inhalation means from each gas-containing container.

The administration period of the pharmaceutical composition is not particularly limited as long as the period allows the pharmaceutical composition to exert the effect of improving prognosis. Those skilled in the art can appropriately set the administration period to a proper period according to the severity of the patient, the cardiopulmonary arrest duration, the time from the return of spontaneous circulation to the start of administration, the type of the disease responsible for cardiac arrest, sex, age, etc. Such a period can be, but is not limited to, for example, at least 1 hour, at least 3 hours, at least 6 hours, at least 9 hours, at least 12 hours, at least 15 hours, at least 18 hours, at least 21 hours, at least 24 hours, at least 2 days, at least 5 days, at least 10 days, at least 15 days, at least 20 days, at least 25 days, or at least 30 days.

The pharmaceutical composition for use of the present invention is not limited by the number of doses and can be administered at a plurality of times. The dose interval and the number of doses of the pharmaceutical composition can be appropriately set to a proper dose interval and number of doses according to the symptoms of the patient.

The pharmaceutical composition for use of the present invention may be used in combination with an additional effective treatment for improvement of prognosis after return of spontaneous circulation. Examples of such a treatment can include, but are not limited to, therapeutic hypothermia.

The pharmaceutical composition for use of the present invention can significantly improve prognosis after return of spontaneous circulation even in a human patient after a lapse of a considerable period of time from the return of spontaneous circulation following cardiopulmonary arrest. For example, as shown below in Examples, the pharmaceutical composition for use in accordance with the present invention improved brain function to attain CPC1 (good cerebral performance) at 90 days after the return of spontaneous circulation for all surviving human patients in a coma having a Glasgow Coma Scale (GCS; well known to those skilled in the art as a 15-point scale for evaluating the severity of impaired consciousness) score of 8 or lower. This indicates that the pharmaceutical composition can improve prognosis very favorably even in a human patient having a considerably low level of consciousness at the time of administration after return of spontaneous circulation.

The terms used in the present specification are given for illustrating particular embodiments and are not intended to limit the invention.

The term "comprising" used in the present specification means that described items (members, steps, factors, numbers, etc.) are present and the presence of the other items (members, steps, factors, numbers, etc.) is not excluded therefrom, unless the context evidently requires different interpretation.

Hereinafter, the present invention will be more specifically described with reference to Examples. However, the present invention is not limited by Examples given below by any means.

### [Examples]

### Hydrogen inhalation test on subject with post-cardiac arrest syndrome (PCAS)

### 1. Test subject

In this test, the selected subjects were patients who satisfied particular criteria: the patients were each raced to a hospital due to out-of-hospital cardiac arrest, resuscitated by return of spontaneous circulation during the emergency transport or in an emergency room, particularly aged 20 to 80, and in a coma with a GCS score of 8 or lower in terms of the level of consciousness at the start of inhalation.

In this clinical trial, the patients after return of spontaneous circulation were in a coma, and it was impossible to obtain their own consent. Therefore, among the patients that satisfied the criteria described above, 5 patients whom spouse, adult child, parent or adult sib provided consent were used as test subjects in the inhalation of hydrogen-supplemented oxygen. Table 2 shows the details of the 5 test subjects.

**[Table 2]**

| Patient | Age | Sex | Initial waveform | Putative cardiac arrest duration (min) | Diagnosis |
|---|---|---|---|---|---|
| 1 | 65 | Female | Asystole | 39 | Pneumonia |
| 2 | 77 | Male | VF | 15 | Hyperkalemia |
| 3 | 47 | Male | VF | 16 | AMI |
| 4 | 80 | Female | VF | 14 | HOCM |
| 5 | 57 | Male | VF | 24 | AMI |

HOCM: hypertrophic obstructive cardiomyopathy
AMI: acute myocardial infarction

### 2. Test drug

In this test, the hydrogen gas inhalation of the test subjects was performed using hydrogen-mixed nitrogen (4% hydrogen and 96% nitrogen) contained on the inspiration side of an existing mechanical ventilator, together with oxygen (i.e., the composition of the hydrogen-supplemented oxygen was as follows: H₂: 2%, O₂: 50%, N₂: 48%). There is no previous report on hydrogen gas inhalation in humans. For sufficient consideration given to its safety, the present inventors tested this time the safety of a hydrogen gas administration method from the side tube of the mechanical ventilator, and necessary amounts of the gases supplied, and confirmed that this test was executable safely for humans.

### 3. Administration

By consent of test subject's spouse, adult child, parent or adult sib, the administration of the hydrogen-supplemented oxygen was started as quickly as possible under temperature control treatment after entrance into a general intensive care unit, and continued to 18 hours later under a doctor's monitoring.

The time from the return of spontaneous circulation to the hydrogen-supplemented oxygen was as shown in Table 3 below.

**[Table 3]**

| Patient | Time from return of spontaneous circulation to start of administration |
|---|---|
| 1 | 3 hours and 41 minutes |
| 2 | 7 hours and 2 minutes |
| 3 | 5 hours and 23 minutes |
| 4 | 4 hours and 56 minutes |
| 5 | 3 hours and 47 minutes |

### 4. Evaluation item

The efficacy of the hydrogen gas inhalation was evaluated on the basis of survival at day 90 of disease and the ratio of good cerebral performance (CPC1) conforming to the Glasgow-Pittsburg Cerebral Performance Category (CPC).

### 5. Results

Table 4 shows the survival rate and CPC1 ratio of the test subjects (hydrogen inhalation group) at day 90 of disease. Table 5 shows the average ages of the hydrogen inhalation group and a control. The control refers to patients who did not inhale the hydrogen-supplemented oxygen among the patients selected according to the paragraph 1.

**[Table 4]**

| | Control N=6 (%) | Hydrogen inhalation N=5(%) | P value |
|---|---|---|---|
| 90 Days Survival | 5 (83) | 4 (80) | 0.73 |
| 90 Days CPC 1 | 3(50) | 4 (80) | 0.35 |

**[Table 5]**

| | Control N=6 (%) | Hydrogen inhalation N=5(%) | P value |
|---|---|---|---|
| Age, y/o* | 43 (13) | 65 (14) | 0.03 |

| | | | |
|---|---|---|---|
| *mean (SD) | | | |

According to the report released by the Fire and Disaster Management Agency of Japan on the survival rate, etc. of patients who suffer cardiopulmonary arrest, the 1-month survival rate of survivors of cardiogenic arrest of cardiopulmonary function tends to decrease with age irrespective of sex, and significantly decreases in 70-year-old or older patients (http://www.fdma.go.jp/neuter/topics/houdou/h21/2101/2101 22-1houdou_h.pdf, Attachment 1-2). Likewise, according to the report, the rehabilitation rate after 1 month of the survivors of cardiogenic arrest of cardiopulmonary function tends to decrease with age irrespective of sex, and significantly decreases in 60-year-old or older patients.

The survival rate of the patients at day 90 of disease was 83% for the control who did not inhale hydrogen, whereas the survival rate was 80% for the hydrogen inhalation group. Thus, almost equal outcomes were obtained. As for the ratio of CPC1, the hydrogen inhalation group exhibited a higher percentage (80% vs 50%). The average age of the patients was higher by 20 years or more in the hydrogen inhalation group than in the control, and the average age of the hydrogen inhalation group was 65 years old. In light of these facts, the results obtained about the improvement of survival rates and brain function by the hydrogen inhalation indicate the significant effect of the hydrogen inhalation.

More surprisingly, the outcomes described above were brought about by the hydrogen inhalation performed after a lapse of at least 3 hours from the return of spontaneous circulation in the test subjects. As already mentioned, reactive oxygen species responsible for multiple organ damage are considered to be generated several minutes after return of spontaneous circulation. Therefore, in such a case where a considerable period of time has passed after return of spontaneous circulation, multiple organ damage ascribable to reactive oxygen species has already progressed. Hence, it is thought that the effect brought about by the removal of reactive oxygen species by hydrogen gas cannot be expected. Thus, the outcomes described above cannot be explained by current technical common sense and are significant, suggesting that there exists another action different from the removal of reactive oxygen species by hydrogen gas.

### [Industrial Applicability]

In accordance with the claims, the gaseous pharmaceutical composition comprising hydrogen gas for use of the present invention can improve prognosis after return of spontaneous circulation even after a lapse of a considerable period of time from the return of spontaneous circulation.

## Claims

1. A gaseous pharmaceutical composition comprising hydrogen gas for use in treating a human patient who has experienced a cardiopulmonary arrest for at least 10 minutes, wherein the pharmaceutical composition is administered to the patient after a lapse of 30 minutes or longer from the return of spontaneous circulation following the cardiopulmonary arrest,
wherein
the prognosis after the return of spontaneous circulation is improved in the human patient with the administration of the pharmaceutical composition as compared with a human patient group without the administration of the pharmaceutical composition, and wherein
the improvement of prognosis is an improvement of brain function.

2. The pharmaceutical composition for use according to claim 1, wherein
the human subject is a subject having a Glasgow Coma Scale (GCS) score of 8 or lower.

3. The pharmaceutical composition for use according to claim 1 or 2, wherein
the "improvement of prognosis after return of spontaneous circulation" is improvement at at least 90 days after the return of spontaneous circulation.

4. The pharmaceutical composition for use according to any one of claims 1 to 3,
wherein
the "improvement of prognosis after return of spontaneous circulation" is determined on the basis of a Cerebral Performance Category (CPC) score.

5. The pharmaceutical composition for use according to any one of claims 1 to 4,
wherein
the pharmaceutical composition is continuously administered for at least 18 hours from the start of administration.

6. The pharmaceutical composition for use according to any one of claims 1 to 5,
wherein
the pharmaceutical composition further comprises oxygen gas.

7. The pharmaceutical composition for use according to any one of claims 1 to 6,
wherein
the pharmaceutical composition further comprises an inert gas.

8. The pharmaceutical composition for use according to any one of claims 1 to 7,
wherein
the hydrogen concentration in the pharmaceutical composition is 0.1% to 4.0%, optionally wherein
the hydrogen concentration in the pharmaceutical composition is 1.0% to 2.0%.

9. The pharmaceutical composition for use according to any one of claims 1 to 8,
wherein
the cardiopulmonary arrest is caused by acute myocardial infarction, cardiomyopathy or hyperkalemia.

10. The pharmaceutical composition for use according to any one of claims 1 to 9,
wherein
the hydrogen gas is provided by a hydrogen gas generation apparatus or a container containing the hydrogen gas.

11. The pharmaceutical composition for use according to claim 10, wherein
the hydrogen gas generation apparatus comprises a hydrogen generation means based on water electrolysis or wherein the container contains a mixed gas of the hydrogen gas and nitrogen gas.

12. The pharmaceutical composition for use according to claim 10 or claim 11, wherein
the hydrogen gas generation apparatus or the container constitutes a device for gas inhalation, together with piping connected to the apparatus or the container and an inhalation means for gas inhalation connected to the piping.

13. The pharmaceutical composition for use according to claim 12, wherein
the device for gas inhalation further comprises an oxygen gas generation apparatus or a container containing oxygen gas.

14. The pharmaceutical composition for use according to claim 12 or 13, wherein
the device for gas inhalation further comprises a means which controls a gas flow rate to the inhalation means.

## Patentansprüche

1. Gasförmige pharmazeutische Zusammensetzung umfassend Wasserstoffgas zur Verwendung bei der Behandlung eines humanen Patienten, der einen kardiopulmonalen Stillstand von mindestens 10 Minuten erlebt hat, wobei die pharmazeutische Zusammensetzung dem Patienten nach einem Ablauf von 30 Minuten oder länger nach Rückkehr des spontanen Kreislaufs nach dem kardiopulmonalen Stillstand verabreicht wird, wobei
die Prognose nach der Rückkehr des spontanen Kreislaufs bei dem humanen Patienten mit der Verabreichung der pharmazeutischen Zusammensetzung im Vergleich zu einer humanen Patientengruppe ohne die Verabreichung der pharmazeutischen Zusammensetzung verbessert ist und wobei die Verbesserung der Prognose eine Verbesserung der Gehirnfunktion ist.

2. Pharmazeutische Zusammensetzung zur Verwendung nach Anspruch 1, wobei das humane Subjekt ein Subjekt mit einer Glasgow-Koma-Skala (GCS) Punktzahl von 8 oder weniger aufweist.

3. Pharmazeutische Zusammensetzung zur Verwendung nach Anspruch 1 oder 2, wobei die "Verbesserung der Prognose nach Rückkehr des spontanen Kreislaufs" eine Verbesserung an mindestens 90 Tagen nach der Rückkehr des spontanen Kreislaufs ist.

4. Pharmazeutische Zusammensetzung zur Verwendung nach einem der Ansprüche 1 bis 3, wobei die "Verbesserung der Prognose nach Rückkehr des spontanen Kreislaufs" auf der Basis einer zerebralen Leistungskategorie- (CPC) Punktzahl bestimmt ist.

5. Pharmazeutische Zusammensetzung zur Verwendung nach einem der Ansprüche 1 bis 4, wobei die pharmazeutische Zusammensetzung ab Beginn der Verabreichung mindestens 18 Stunden lang kontinuierlich verabreicht wird.

6. Pharmazeutische Zusammensetzung zur Verwendung nach einem der Ansprüche 1 bis 5, wobei die pharmazeutische Zusammensetzung ferner Sauerstoffgas umfasst.

7. Pharmazeutische Zusammensetzung zur Verwendung nach einem der Ansprüche 1 bis 6. wobei die pharmazeutische Zusammensetzung ferner ein Inertgas umfasst.

8. Pharmazeutische Zusammensetzung zur Verwendung nach einem der Ansprüche 1 bis 7, wobei die Wasserstoffkonzentration in der pharmazeutischen Zusammensetzung 0,1% bis 4,0% beträgt, gegebenenfalls wobei die Wasserstoffkonzentration in der pharmazeutischen Zusammensetzung 1,0% bis 2,0% beträgt.

9. Pharmazeutische Zusammensetzung zur Verwendung nach einem der Ansprüche 1 bis 8, wobei der kardiopulmonale Stillstand durch akuten Myokardinfarkt, Kardiomyopathie oder Hyperkaliämie verursacht ist.

10. Pharmazeutische Zusammensetzung zur Verwendung nach einem der Ansprüche 1 bis 9, wobei das Wasserstoffgas durch eine Wasserstoffgaserzeugungsvorrichtung oder einen Behälter, der das Wasserstoffgas enthält, bereitgestellt ist.

11. Pharmazeutische Zusammensetzung zur Verwendung nach Anspruch 10, wobei die Wasserstoffgaserzeugungsvorrichtung ein Wasserstofferzeugungsmittel umfasst, das auf Wasserelektrolyse basiert oder wobei der Behälter ein Mischgas aus Wasserstoffgas und Stickstoffgas enthält.

12. Pharmazeutische Zusammensetzung zur Verwendung nach Anspruch 10 oder Anspruch 11, wobei die Wasserstoffgaserzeugungsvorrichtung oder der Behälter zusammen mit einer mit der Vorrichtung oder dem Behälter verbundenen Rohrleitung und einem Inhalationsmittel zur Gasinhalation, das mit der Rohrleitung verbunden ist, eine Vorrichtung zur Gasinhalation darstellt.

13. Pharmazeutische Zusammensetzung zur Verwendung nach Anspruch 12, wobei die Vorrichtung zur Gasinhalation ferner eine Sauerstoffgaserzeugungsvorrichtung oder einen Behälter, der Sauerstoffgas enthält, umfasst.

14. Pharmazeutische Zusammensetzung zur Verwendung nach Anspruch 12 oder 13, wobei die Vorrichtung zur Gasinhalation ferner ein Mittel umfasst, das eine Gasflussrate zum Inhalationsmittel steuert.

## Revendications

1. Composition pharmaceutique gazeuse comprenant de l'hydrogène gazeux pour utilisation dans le traitement d'un patient humain qui a subi un arrêt cardio-pulmonaire pendant au moins 10 minutes, où la composition pharmaceutique est administrée au patient après un laps de temps de 30 minutes ou plus à partir du retour de la circulation spontanée après l'arrêt cardio-pulmonaire, où
le pronostic après le retour de la circulation spontanée est amélioré chez le patient humain avec l'administration de la composition pharmaceutique par rapport à un groupe de patients humains sans l'administration de la composition pharmaceutique, et où
l'amélioration du pronostic est une amélioration de la fonction cérébrale.

2. Composition pharmaceutique pour utilisation selon la revendication 1, où
le sujet humain est un sujet ayant un score de l'Échelle de Glasgow (GCS) inférieur ou égal à 8.

3. Composition pharmaceutique pour utilisation selon la revendication 1 ou 2, où
"l'amélioration du pronostic après le retour de la circulation spontanée" est une amélioration au moins 90 jours après le retour de la circulation spontanée.

4. Composition pharmaceutique pour utilisation selon l'une quelconque des revendications 1 à 3, où
"l'amélioration du pronostic après le retour de la circulation spontanée" est déterminée sur la base d'un score de Catégorie de Performance Cérébrale (CPC).

5. Composition pharmaceutique pour utilisation selon l'une quelconque des revendications 1 à 4, où
la composition pharmaceutique est administrée en continu pendant au moins 18 heures depuis le début de l'administration.

6. Composition pharmaceutique pour utilisation selon l'une quelconque des revendications 1 à 5, où
la composition pharmaceutique comprend en outre de l'oxygène gazeux.

7. Composition pharmaceutique pour utilisation selon l'une quelconque des revendications 1 à 6, où
la composition pharmaceutique comprend en outre un gaz inerte.

8. Composition pharmaceutique pour utilisation selon l'une quelconque des revendications 1 à 7, où
la concentration d'hydrogène dans la composition pharmaceutique est de 0,1 % à 4,0 %, facultativement où
la concentration d'hydrogène dans la composition pharmaceutique est de 1,0 % à 2,0 %.

9. Composition pharmaceutique pour utilisation selon l'une quelconque des revendications 1 à 8, où
l'arrêt cardio-pulmonaire est causé par un infarctus aigu du myocarde, une cardiomyopathie ou une hyperkaliémie.

10. Composition pharmaceutique pour utilisation selon l'une quelconque des revendications 1 à 9, où
l'hydrogène gazeux est fourni par un appareil de génération d'hydrogène gazeux ou un récipient contenant l'hydrogène gazeux.

11. Composition pharmaceutique pour utilisation selon la revendication 10, où
l'appareil de génération d'hydrogène gazeux comprend un moyen de génération d'hydrogène basé sur l'électrolyse de l'eau ou où le récipient contient un mélange gazeux d'hydrogène gazeux et d'azote gazeux.

12. Composition pharmaceutique pour utilisation selon la revendication 10 ou la revendication 11, où
l'appareil de génération d'hydrogène gazeux ou le récipient constitue un dispositif pour l'inhalation de gaz, conjointement avec une tuyauterie reliée à l'appareil ou au récipient et un moyen d'inhalation pour l'inhalation de gaz relié à la tuyauterie.

13. Composition pharmaceutique pour utilisation selon la revendication 12, où
le dispositif pour l'inhalation de gaz comprend en outre un appareil de génération d'oxygène gazeux ou un récipient contenant de l'oxygène gazeux.

14. Composition pharmaceutique pour utilisation selon la revendication 12 ou 13, où
le dispositif pour l'inhalation de gaz comprend en outre un moyen qui régule un débit de gaz vers le moyen d'inhalation.
